# EUROPEAN PATENT SPECIFICATION

(11) **EP 3 936 576 B1**
(45) Date of publication and mention of the grant of the patent: **28.05.2025**
(21) Application number: 20767251.0
(22) Date of filing: 04.03.2020
(51) Int. Cl.: C09C 1/64, C09C 3/06, A61K 8/02, A61K 8/25, A61K 8/26, A61K 8/29, C08K 9/02, C09C 3/00, C09C 3/10, C09D 7/61, C09D 7/62, C09D 7/40

(54) **BLACK ALUMINUM PIGMENT AND METHOD OF PRODUCING SAME**
SCHWARZES ALUMINIUMPIGMENT UND VERFAHREN ZU SEINER HERSTELLUNG
PIGMENT D'ALUMINIUM NOIR ET SON PROCÉDÉ DE PRODUCTION

(30) Priority: 05.03.2019 JP 2019039186
(43) Date of publication of application: 12.01.2022
(73) Proprietor: Toyo Aluminium Kabushiki Kaisha, Osaka-shi, Osaka 541-0056 (JP); Ako Kasei Co., Ltd., Ako-shi, Hyogo 678-0193 (JP)
(72) Inventor: KAWASHIMA, Katsura, Osaka-shi, Osaka 541-0056 (JP); NAGANO, Keita, Osaka-shi, Osaka 541-0056 (JP); SETOGUCHI, Shunichi, Osaka-shi, Osaka 541-0056 (JP); UOZUMI, Yoshinobu, Ako-shi, Hyogo 678-0193 (JP); NAKAHARA, Tomomi, Ako-shi, Hyogo 678-0193 (JP)
(74) Representative: Bockhorni & Brüntjen Partnerschaft Patentanwälte mbB
(86) International application number: PCT/JP2020/009244
(87) International publication number: WO 2020/179840

(56) References cited:
- EP-A1- 1 114 103
- EP-A2- 2 686 386
- WO-A2-2006/131472
- JP-A- 2006 509 088
- JP-A- 2008 545 866
- JP-A- 2010 185 073
- JP-A- H01 311 176
- JP-A- H08 209 024

## Description

### Technical Field

The present invention relates to a black aluminum pigment and a method of producing the same.

### BACKGROUND ART

Conventionally, pigments that provide a variety of color tones are known. For example, in Japanese Patent Laying-Open No. 2010-185073 (Patent Literature 1), a dichroic pigment in which a single layer of lower titanium oxide is formed on the surface of mica is disclosed.
WO 2006/131472 A2 discloses a process for the treatment of particles using a plasma torch and to the particles obtainable by the process, in particular flakes having a metal core (e.g., aluminum) followed by a layer of metal oxide and a semi-transparent coating, wherein the coating film is layered by an amorphous silicon compound layer and a titanium oxide layer in this order.
EP 1 114 103 A1 discloses multi layer pigments based on coated metal lamina. EP 2 686 386 A2 discloses black effect pigments.

### CITATION LIST

### PATENT LITERATURE

PTL 1: Japanese Patent Laying-Open No. 2010-185073

### SUMMARY OF INVENTION

### TECHNICAL PROBLEM

In recent years, various design properties are required of pigments, and there are pigments that provide a black color having a metallic appearance as one of the various design properties (hereinafter also referred to as "brilliant black pigments"). The brilliant black pigments can provide sharp design properties in paints, cosmetics, and the like. It is an object of the present invention to provide a black aluminum pigment that can be utilized as a brilliant black pigment, and a method of producing the same.

### SOLUTION TO PROBLEM

The present invention is as follows.

A black aluminum pigment includes a flaky aluminum particle; and a coating film that covers the aluminum particle, wherein the coating film includes a titanium oxide layer and an amorphous silicon compound layer, wherein the titanium oxide layer and the amorphous silicon compound layer are layered in this order, the titanium oxide layer has a composition that satisfies TiOₓ (0.50 ≤ x ≤ 1.90), and the amorphous silicon compound layer is composed of at least one of silicon oxide, silicon hydroxide, and silicon hydrate.

In one embodiment, the titanium oxide layer has a thickness of 50 nm or more and 1000 nm or less.

In one embodiment, the amorphous silicon compound layer has a thickness of 10 nm or more and 1000 nm or less.

A method of producing the black aluminum pigment includes preparing an aluminum particle; and forming a coating film on the aluminum particle, wherein forming the coating film has forming a titanium oxide layer, and forming an amorphous silicon compound layer, when the amorphous silicon compound layer is interposed between the aluminum particle and the titanium oxide layer, the titanium oxide layer is formed by subjecting to reduction treatment a titanium dioxide layer formed by hydrolysis treatment, or when the amorphous silicon compound is not interposed between the aluminum particle and the titanium oxide layer, the titanium oxide layer is formed by subjecting to reduction treatment a titanium dioxide layer formed by sol-gel treatment.

### ADVANTAGEOUS EFFECTS OF INVENTION

According to the present invention, a black aluminum pigment that can be utilized as a brilliant black pigment, and a method of producing the same can be provided.

### BRIEF DESCRIPTION OF DRAWINGS

FIG. 1 is a schematic cross-sectional view of a black aluminum pigment according to an embodiment.
FIG. 2 is a schematic cross-sectional view of a black aluminum pigment in accordance with the invention, when it has a coating film in which a titanium oxide layer and an amorphous silicon compound layer are layered in this order from the aluminum particle side.
FIG. 3 is a schematic cross-sectional view of a black aluminum pigment not in accordance with the invention, when it has a coating film in which an amorphous silicon compound layer and a titanium oxide layer are layered in this order from the aluminum particle side.
FIG. 4 is a schematic cross-sectional view of a black aluminum pigment when it has a coating film in which an amorphous silicon compound layer, a titanium oxide layer, and an amorphous silicon compound layer are layered in this order from the aluminum particle side.
FIG. 5 is a diagram showing the analysis results of powder X-ray diffraction for the black aluminum pigments of Examples 1 to 4 and the aluminum pigment of Comparative Example 1.

### DESCRIPTION OF EMBODIMENTS

A black aluminum pigment and a method of producing the same according to this embodiment will each be described in detail below. In the drawings used for the following description of embodiments, identical reference numerals indicate identical parts or corresponding parts. However, in all the following drawings, components are shown by appropriately adjusting dimensional relationships for easy understanding, and the scales of the components shown in the drawings and the dimensional relationships of actual components are not necessarily the same. As used herein, the expression of the form "A to B" means the upper limit and lower limit of a range (that is, A or more and B or less), and when there is no description of a unit for A, and a unit is described only for B, the unit of A and the unit of B are the same.

### [Black Aluminum Pigment]

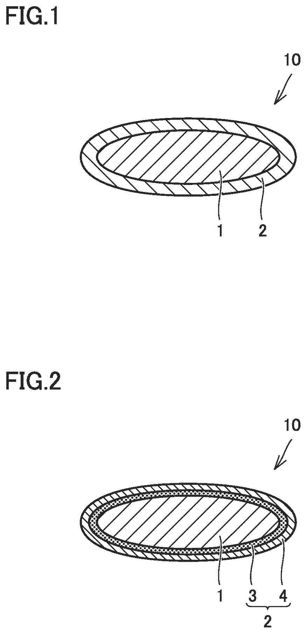

With reference to FIG. 1 to FIG. 4, a black aluminum pigment 10 includes a flaky aluminum particle 1 and a coating film 2 that covers aluminum particle 1. Coating film 2 includes a titanium oxide layer 3 and an amorphous silicon compound layer 4. Titanium oxide layer 3 has a composition that satisfies TiOₓ (0.50 ≤ x ≤ 1.90), and amorphous silicon compound layer 4 is composed of at least one of silicon oxide, silicon hydroxide, and silicon hydrate.

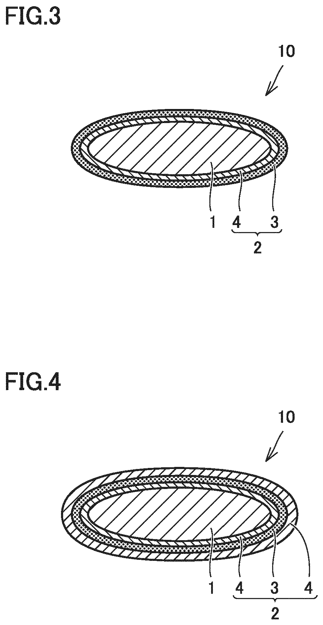

Coating film 2 includes at least one titanium oxide layer 3 and at least one amorphous silicon compound layer 4. As long as the effect of the present invention is achieved and a titanium oxide layer and an amorphous silicon compound layer are layered in this order from the aluminum particle side, the positions of the layers and the numbers of layers are not particularly limited. For example, in FIG. 2, coating film 2 in which one titanium oxide layer 3 and one amorphous silicon compound layer 4 are layered in this order from the aluminum particle 1 side is shown; in FIG. 3, not in accordance with the invention, coating film 2 in which one amorphous silicon compound layer 4 and one titanium oxide layer 3 are layered in this order from the aluminum particle 1 side is shown; and in FIG. 4, a coating film 2 in which one amorphous silicon compound layer 4, one titanium oxide layer 3, and another amorphous silicon compound layer 4 are layered in this order from the aluminum particle 1 side is shown. Coating film 2 may include other layers other than titanium oxide layer 3 and amorphous silicon compound layer 4. Examples of preferred other layers include a base layer and a resin layer.

Black aluminum pigment 10 can be utilized as a brilliant black pigment. In other words, black aluminum pigment 10 can provide a black color having a glittering metallic appearance. In black aluminum pigment 10, brilliance is exhibited by aluminum particle 1 that is a base material, and a black color is provided by titanium oxide layer 3. For providing a black color, not only when a color tone is visually recognized as black in visual inspection, but also when a color tone is visually recognized as bluish black or reddish black in visual inspection, the color tone is determined to provide a black color when the L value is 30 or less.

Here, the L value is a value that represents lightness in the Hunter color system. Colorimetry is performed by a powder cell method. Specifically, 1 g of a (black) aluminum pigment is added to a glass cell, and a surface pressure of 25.5 g/cm² is applied to form a measurement sample. For the conditions of the colorimetry, D/0° is set, and a C light source is adopted.

The shape of black aluminum pigment 10 depends mainly on the shape of aluminum particle 1 because coating film 2 generally evenly covers the entire surface of aluminum particle 1 so as to conform to the shape of aluminum particle 1. Even if there are irregularities on the surface of coating film 2, the overall shape characteristics of black aluminum pigment 10 do not change due to the irregularities, considering the relationship between the size of aluminum particle 1 and the thickness of coating film 2. Therefore, the shape of black aluminum pigment 10 is flaky, that is, a scale-like shape.

The average particle diameter (D50) of black aluminum pigment 10 is preferably 1 to 300 µm, more preferably 5 to 30 µm. When D50 is 1 µm or more, a good metallic finished appearance can be given to a coating (a film including black aluminum pigment 10), and high hiding power can be exhibited. When D50 is 300 µm or less, the dispersibility of black aluminum pigment 10 in the coating is good. D50 can be measured using a particle diameter distribution measuring apparatus using a laser diffraction scattering method as a measurement principle. Here, D50 means that the volume occupied by particles having a particle diameter represented by D50, or less, is 50% of the overall volume.

The average thickness of black aluminum pigment 10 is preferably 0.01 to 5 µm. In this case, a good appearance can be given while the light resistance and weather resistance of the coating are maintained high. The average thickness is more preferably 0.015 to 3 µm. The average thickness can be obtained, for example, by observing black aluminum pigment 10 using a scanning electron microscope (SEM), a transmission electron microscope (TEM), and the like, and calculating the average value of 500 or more.

### <Aluminum Particle>

Aluminum particle 1 is an aluminum particle that is flaky, that is, has a scale-like shape. This aluminum particle may be composed of pure aluminum or an aluminum alloy. Pure aluminum is aluminum (Al) having a purity of 99.7% by mass or more, and an aluminum alloy is an alloy including Al as a main component. Specific examples of the aluminum alloy include 1000 to 8000 series aluminum alloys and these aluminum alloys to which other elements other than Al are added. Preferred other elements are silicon (Si), zinc (Zn), chromium (Cr), manganese (Mn), magnesium (Mg), copper (Cu), and the like.

The total amount of the components other than Al blended in the aluminum alloy is preferably less than 50% by mass based on 100% by mass of the aluminum alloy. For example, the blending proportion of Si is preferably 40% by mass or less based on 100% by mass of the aluminum alloy, and the blending proportion of Mg is preferably 10% by mass or less. The metal components included in aluminum particle 1 can be quantified by inductively coupled plasma (ICP) atomic emission spectroscopy.

The average particle diameter (D50) of aluminum particles 1 is preferably 1 to 300 µm, more preferably 5 to 30 µm. When D50 is 1 µm or more, a good metallic finished appearance can be given to a coating (a film including black aluminum pigment 10), and high hiding power can be exhibited. When D50 is 300 µm or less, the dispersibility of black aluminum pigment 10 in the coating is good. When black aluminum pigment 10 has a scale-like shape, the D50 of aluminum particles 1 is generally the same as the D50 of black aluminum pigment 10.

The average thickness of aluminum particles 1 is preferably 0.01 to 5 µm. In this case, a good appearance can be given while the light resistance and weather resistance of the coating are maintained high. The average thickness is more preferably 0.015 to 1 µm. The average thickness of aluminum particles 1 can be obtained, for example, by performing SEM observation or TEM observation on a specimen including a cross section of black aluminum pigment 10 (cross-sectional specimen), measuring the thickness of aluminum particles 1 of 500 black aluminum pigments 10, and calculating its average value. Examples of the cross-sectional specimen include a specimen obtained by slicing a resin lump to which black aluminum pigment 10 is fixed.

For raw aluminum particles 1 not covered with coating films 2, the D50 and average thickness of the aluminum particles can be calculated by using the above laser diffraction scattering method using a particle diameter distribution measuring apparatus, and a water surface diffusion area method.

### <Coating Film>

Coating film 2 covers aluminum particle 1. As described above, coating film 2 includes at least one titanium oxide layer 3 and at least one amorphous silicon compound layer 4, and as long as the effect of the present invention is achieved and a titanium oxide layer and an amorphous silicon compound layer are layered in this order from the aluminum particle side, the positions of the layers and the numbers of layers are not particularly limited, and coating film 2 may include other layers (not shown).

**In** FIG. 1 to FIG. 4, cases where coating film 2 uniformly covers all the surface of aluminum particle 1 are illustrated, but the shape of coating film 2 is not limited to this. As long as the effect of the present invention is achieved, part of aluminum particle 1 may be exposed, and the thickness of coating film 2 may be nonuniform. However, from the viewpoint of providing high brilliance and a homogeneous black color, coating film 2 preferably uniformly covers all the surface of aluminum particle 1.

The thickness of coating film 2 is preferably 20 to 2000 nm, more preferably 40 to 1000 nm. When the thickness is less than 20 nm, the thickness of titanium oxide layer 3 and amorphous silicon compound layer 4 is too thin, and thus the effect of coating film 2 described later may be insufficient. When the thickness exceeds 2000 nm, the hiding power of black aluminum pigment 10 per unit weight decreases, and thus the commercial value may decrease. In addition, there is a possibility that brilliance by aluminum particle 1 cannot be sufficiently exhibited.

The thickness of coating film 2 can be obtained by performing SEM observation or TEM observation on a specimen including a cross section of black aluminum pigment 10 (cross-sectional specimen), measuring the thickness of coating films 2 of 500 black aluminum pigments 10, and calculating its average value. When the thickness of coating film 2 is nonuniform, thickness at any 10 points is measured in each black aluminum pigment 10, and its average value is taken as the thickness of coating film 2 of each black aluminum pigment 10. The 10 points at this time needs to be extracted so that a suitable average value of the thickness is calculated Examples of the cross-sectional specimen include a specimen obtained by slicing a resin lump to which black aluminum pigment 10 is fixed.

### <Titanium Oxide Layer>

Titanium oxide layer 3 has a composition that satisfies TiOₓ (0.50 ≤ x ≤ 1.90). Thus, titanium oxide layer 3 itself can provide a black color. In addition, titanium oxide layer 3 does not hide the brilliance of aluminum particle 1. In other words, titanium oxide layer 3 is a layer that provides a black color without completely hiding the base. The X value of TiOₓ more preferably satisfies 1.00 ≤ x ≤ 1.90, further preferably 1.10 ≤ x ≤ 1.90.

The composition of titanium oxide layer 3 is determined by observing titanium oxide layer 3 using a powder diffraction X-ray analysis apparatus, and performing quantitative analysis using an RIR (Reference Intensity Ratio) method. For example, when the RIR method is used for the analysis of the composition of titanium oxide layer 3 using a powder diffraction X-ray analysis apparatus, and Ti₃O₅ and Ti₄O₇ are confirmed, and the respective composition proportions are analyzed as 15% by mass and 85% by mass from quantitative analysis, value X of the amount of oxygen, per the basis of a titanium atom formed in titanium oxide layer 3 (TiOₓ) is calculated from the amounts of oxygen and composition proportions of the compositions. In this case, for example, the amounts of oxygen per the basis of a titanium atom for Ti₃O₅ and Ti₄O₇ are 26.7 and 28.0 respectively. By converting the amounts of oxygen by the composition proportions based on these numerical values, and dividing the converted value by oxygen atomic weight, 15.99, the X value is calculated. This calculation formula is ((26.7) × 0.15 + (28.0) × 0.85)/15.99 = 1.74. When a plurality of titanium oxide layers 3 are present in coating film 2, the X value calculated by the above method is the averaged value of the compositions of the plurality of titanium oxide layers 3.

The thickness of titanium oxide layer 3 is preferably 50 to 1000 nm, more preferably 100 to 600 nm. When the thickness is less than 50 nm, the absorption of visible light by titanium oxide layer 3 is insufficient, and as a result, it may be difficult to provide a black color having high design properties. When the thickness exceeds 1000 nm, the absorption of visible light by titanium oxide layer 3 is excessive, and metallic gloss by aluminum particle 1 may not be exhibited.

The thickness of titanium oxide layer 3 can be obtained by performing SEM observation or TEM observation on a cross-sectional specimen of black aluminum pigment 10, measuring the thickness of titanium oxide layers 3 of 100 black aluminum pigments 10, and calculating its average value. When the thickness of any one titanium oxide layer 3 is nonuniform, thickness at any 10 or more points is measured in the titanium oxide layer 3, and its average value is taken as the thickness of the titanium oxide layer 3. When a plurality of titanium oxide layers 3 are present in coating film 2, the total of the thickness of all titanium oxide layers 3 is regarded as "the thickness of the titanium oxide layer".

In FIG. 2 to FIG. 4, cases where titanium oxide layer 3 uniformly covers the entire aluminum particle 1 are illustrated, but the shape of titanium oxide layer 3 is not limited to this. As long as the effect of the present invention is achieved, part may be missing, and its thickness may be nonuniform. However, from the viewpoint of providing high brilliance and a homogeneous black color, titanium oxide layer 3 preferably has uniform thickness and is preferably a continuous layer that uniformly covers all aluminum particle 1.

When a plurality of titanium oxide layers 3 are present in coating film 2, each composition of each titanium oxide layer 3 needs to satisfy TiOₓ (0.50 ≤ x ≤ 1.90), but the respective compositions may be the same or different. The respective thicknesses may also be the same or different.

### <Amorphous Silicon Compound Layer>

Amorphous silicon compound layer 4 is composed of at least one of silicon oxide, silicon hydroxide, and silicon hydrate. The composition of amorphous silicon compound layer 4 can be confirmed using, for example, EDX (Energy Dispersive X-ray spectrometry). Amorphous silicon compound layer 4 is a layer excellent in water resistance. The "amorphous" of amorphous silicon compound layer 4 means that amorphous silicon compound layer 4 is in a state in which no clear diffraction peaks derived from silicon oxide are detected in crystal structure analysis by an X-ray diffraction method.

Aluminum particle 1 has low water resistance, and therefore when this is used as the base material of the pigment, it is feared that the water resistance of black aluminum pigment 10 is decreased. In addition, depending on the method of forming coating film 2 on aluminum particle 1, aluminum particle 1 dissolves in the process of producing the black aluminum pigment, and a pigment having commercial value may not form. But according to black aluminum pigment 10 according to this embodiment, amorphous silicon compound layer 4 is present in coating film 2, and therefore the water resistance of black aluminum pigment 10 as a whole can be maintained sufficiently high, and the problem in the process of production as described above can be eliminated. When amorphous silicon compound layer 4 includes silicon hydroxide and/or silicon hydrate, the content of these is low to the extent that the water resistance of black aluminum pigment 10 is not impaired.

The thickness of amorphous silicon compound layer 4 is preferably 10 to 1000 nm. In this case, black aluminum pigment 10 can maintain high both characteristics of sufficient water resistance and good hiding power. Here, the hiding power means the hiding power of black aluminum pigment 10 per unit weight. This hiding power decreases as the thickness of coating film 2 increases. When the thickness is less than 10 nm, the water resistance may be insufficient. When the thickness exceeds 1000 nm, the hiding power may decrease.

The thickness of amorphous silicon compound layer 4 can be obtained by performing SEM observation or TEM observation on a cross-sectional specimen of black aluminum pigment 10, measuring the thickness of amorphous silicon compound layers 4 of 500 black aluminum pigments 10, and calculating its average value. When the thickness of any one amorphous silicon compound layer 4 is nonuniform, thickness at any 10 or more points is measured in the amorphous silicon compound layer 4, and its average value is taken as the thickness of the amorphous silicon compound layer 4. When a plurality of amorphous silicon compound layers 4 are present in coating film 2, the total thickness of all amorphous silicon compound layers 4 is regarded as "the thickness of the amorphous silicon compound layer".

In FIG. 2 to FIG. 4, cases where amorphous silicon compound layer 4 uniformly covers the entire aluminum particle 1 are illustrated, but the shape of amorphous silicon compound layer 4 is not limited to this. As long as the effect of the present invention is achieved, part may be missing, and its thickness may be nonuniform. However, from the viewpoint of providing high brilliance and a homogeneous black color while having high water resistance, amorphous silicon compound layer 4 preferably has uniform thickness and is preferably a continuous layer that uniformly covers all aluminum particle 1.

When a plurality of amorphous silicon compound layers 4 are present in coating film 2 as shown in FIG. 4, the compositions of amorphous silicon compound layers 4 may be the same or different. The respective thicknesses may also be the same or different.

### <Base layer>

Examples of preferred other layers included in coating film 2 include a base layer composed of at least one of an oxide, hydroxide, and hydrate of molybdenum (Mo) and/or phosphorus (P). For example, by disposing the base layer on the surface of aluminum particle 1, the water resistance of aluminum particle 1 can be more effectively supplemented. In addition, when other layers grow on the base layer, the base layer can be a good starting point of growth. Particularly, good growth of an amorphous silicon compound is possible. The shape and thickness of the base layer is not particularly limited as long as the effect of the present invention is achieved. The base layer may be a continuous layer or a discontinuous layer.

### <Resin Layer>

Examples of preferred other layers included in coating film 2 include a resin layer. By including the resin layer as the outermost layer of coating film 2, the chemical resistance of black aluminum pigment 10 improves. In addition, in this case, the adhesiveness between black aluminum pigment 10 and a resin increases in a resin composition containing black aluminum pigment 10, and therefore the physical properties of a coating obtained by coating an object to be coated, with the resin composition improve. When coating film 2 includes the resin layer, the amount of the covering resin layer is preferably 0.5 to 100 parts by mass and preferably 1 to 50 parts by mass based on aluminum particle 1. In this case, black aluminum pigment 10 can highly achieve both the function of providing metallic gloss and a black color and the function of chemical resistance.

As the resin preferred for the resin layer, a copolymerized resin obtained by copolymerizing two or more types of polymerizable monomers is preferred. Examples of the polymerizable monomers include reactive monomers having a carboxyl group and/or a phosphate group, tri- or higher polyfunctional acrylic ester monomers, and polymerizable monomers having a benzene nucleus. Specific examples of the monomers will be described later.

### [Method of Producing Black Aluminum Pigment]

The method of producing black aluminum pigment 10 includes the step of preparing aluminum particles (preparation step), and the step of forming coating films on the aluminum particles (coating film forming step). The coating film forming step has the step of forming titanium oxide layers (titanium oxide layer forming step), and the step of forming amorphous silicon compound layers (amorphous silicon compound layer forming step).

### <Preparation Step>

In this step, aluminum particles 1 that are base materials are prepared. Aluminum particles 1 having a flake shape can be made by a conventionally known method. For example, aluminum particles 1 can be made by peeling an aluminum thin film formed on a surface of a plastic film by vapor deposition, from the surface of the plastic film and then crushing the aluminum thin film. In addition, for example, aluminum particles 1 can also be made by grinding aluminum particles obtained using a conventionally known atomization method, in the presence of an organic solvent using a ball mill.

### <Coating Film Forming Step>

In this step, coating films 2 are formed on the prepared aluminum particles 1. This step has a titanium oxide layer forming step and an amorphous silicon compound layer forming step, and the titanium oxide layer forming step differs depending on the disposition state of titanium oxide layers 3 and amorphous silicon compound layers 4 in coating films 2. This step may further include the step of forming base layers (base layer forming step), and/or the step of forming resin layers (resin layer forming step). The steps will be described in detail below.

### <<Titanium Oxide Layer Forming Step>>

In this step, titanium oxide layers 3 are formed on the surfaces of objects to be covered. Regarding this step, when amorphous silicon compound layers 4 are interposed between aluminum particles 1 and titanium oxide layers 3, titanium oxide layers 3 are formed by subjecting to reduction treatment titanium dioxide layers formed by hydrolysis treatment, and when amorphous silicon compound layers 4 are not interposed, titanium oxide layers 3 are formed by subjecting to reduction treatment titanium dioxide layers formed by sol-gel treatment. The method of forming titanium oxide layers 3 in the former case (the case where amorphous silicon compound layers 4 are interposed between aluminum particles 1 and titanium oxide layers 3) will be described below as a first method, and the method of forming titanium oxide layers 3 in the latter case (the case where amorphous silicon compound layers 4 are not interposed between aluminum particles 1 and titanium oxide layers 3) will be described below as a second method.

### (First Method: Case Where Amorphous Silicon Compound Layers 4 Are Interposed)

In the first method, the treatments of the following (1) and (2) are carried out in this order on objects to be covered. The objects to be covered are, for example, particles in which amorphous silicon compound layers 4 are formed on the surfaces of aluminum particles 1.

### (1) Hydrolysis Treatment

In this treatment, by hydrolyzing a titanium salt in water in which objects to be covered are dispersed, titanium dioxide layers are formed on the surfaces of the objects to be covered. Specifically, the objects to be covered are dispersed in water to form a slurry, the pH of the slurry is adjusted to be 1.3 to 5.0, and then a titanium salt aqueous solution is introduced while the pH is kept constant using a basic aqueous solution, to hydrolyze the titanium salt. Thus, titanium dioxide layers having relatively uniform thickness are formed on the surfaces of the objects to be covered. These titanium dioxide layers are continuous layers.

Examples of the titanium salt include titanium tetrachloride and titanyl sulfate. Examples of preferred basic aqueous solutions include aqueous solutions of watersoluble amines, sodium hydroxide, potassium hydroxide, and the like, and ammonia water.

### (2) Reduction Treatment

In this treatment, the titanium dioxide layers formed on the surfaces of the objects to be covered, by the hydrolysis treatment are reduced to change the titanium dioxide layers to titanium oxide layers 3 that satisfy TiOₓ (0.50 ≤ x ≤ 1.90). Specifically, the objects to be covered on which the titanium dioxide layers are formed and a reduction aid are mixed, and this is fired under a reducing atmosphere. The value of X can be adjusted by appropriately changing the firing temperature, the firing time, the reducing atmosphere, and the reduction aid in the reduction treatment.

The firing temperature is preferably 300 to 650°C and preferably 400 to 630°C. When the firing temperature exceeds 650°C, aluminum particles 1 may melt. At a firing temperature of less than 300°C, the time required for the reduction treatment is excessively long, and the production efficiency decreases. In addition, considering that amorphous silicon compound layers 4 are interposed between aluminum particles 1 and titanium dioxide layers, the firing temperature is preferably 300°C or more and less than 500°C because when the firing temperature exceeds 500°C, defects such as cracking and chipping may occur in amorphous silicon compound layers 4.

The firing time is preferably 0.5 to 72 h, more preferably 1 to 24 h. When the firing time is too short, the reduction may be insufficient. When the firing time is too long, the production efficiency decreases. By placing the reduction treatment environment under an atmosphere of a reducing component gas such as nitrogen, hydrogen, ammonia, carbon monoxide, mononitrogen monoxide, dinitrogen monoxide, hydrogen sulfide, or sulfur dioxide or a mixed gas thereof or under vacuum, a preferred reducing atmosphere can be provided.

Examples of the reduction aid include metal titanium, titanium hydride, sodium borohydride, and lithium aluminum hydride. When metal titanium is used as the reduction aid, the firing temperature is preferably 500 to 650°C, and 0.01 to 2.0 mol of metal titanium is preferably used based on 100 g of titanium dioxide. When a hydride such as titanium hydride, sodium borohydride, or lithium aluminum hydride is used as the reduction aid, the amount of the reduction aid added is preferably prepared so that the reducing component gas (H₂) generated when the hydride is decomposed is 0.001 to 30.0 mol based on 100 g of titanium dioxide, and preferably prepared so that the reducing component gas (H₂) is 0.01 to 10.0 mol.

By the above, titanium oxide layers 3 are formed so as to be in contact with the surfaces of the objects to be covered. The first method is preferred when black aluminum pigment 10 in which amorphous silicon compound layers 4 are interposed between aluminum particles 1 and titanium oxide layers 3 as shown in FIG. 3 and FIG. 4 is produced, because amorphous silicon compound layers 4 are excellent in water resistance, and therefore the objects to be covered, that is, "aluminum particles 1 covered with amorphous silicon compound layers 4", can be introduced into water. If hydrolysis treatment is carried out using aluminum particles 1 not covered with amorphous silicon compound layers 4, aluminum particles 1 dissolve while generating hydrogen gas.

### (Second Method: Case Where Amorphous Silicon Compound Layers 4 Are Not Interposed)

In the second method, the treatments of the following (3) and (4) are carried out in this order on objects to be covered. The objects to be covered are, for example, aluminum particles 1.

### (3) Sol-Gel Treatment

In this treatment, titanium dioxide layers composed of titanium dioxide and/or a hydrate thereof are formed on the surfaces of objects to be covered, using a sol-gel method. Specifically, the objects to be covered are dispersed in a hydrophilic organic solvent to form a slurry, and a titanium alkoxide and water are added while the slurry is stirred. Thus, titanium dioxide layers having relatively uniform thickness are formed on the surfaces of the objects to be covered. These titanium dioxide layers are continuous layers.

Examples of the hydrophilic organic solvent include methyl alcohol, ethyl alcohol, isopropyl alcohol, n-propyl alcohol, t-butyl alcohol, n-butyl alcohol, isobutyl alcohol, ethyl cellosolve, butyl cellosolve, propylene glycol monobutyl ether, dipropylene glycol monomethyl ether, propylene glycol monopropyl ether, and acetone. Examples of the titanium alkoxide include isopropoxide, butoxide, octoxide, condensates thereof, or chelate compounds thereof.

### (4) Reduction Treatment

In this treatment, the titanium dioxide layers formed on the surfaces of the objects to be covered, by the sol-gel treatment are reduced to change the titanium dioxide layers to titanium oxide layers 3 that satisfy TiOₓ (0.50 ≤ x ≤ 1.90). The specific treatment method is the same as the above (2), and therefore its description will not be repeated.

By the above, titanium oxide layers 3 are formed so as to be in contact with the surfaces of the objects to be covered. The second method is preferred when black aluminum pigment 10 in which amorphous silicon compound layers 4 are not interposed between aluminum particles 1 and titanium oxide layers 3 as shown in FIG. 2 is produced. According to the second method, homogeneous titanium oxide layers 3 can be simply formed.

### <Amorphous Silicon Compound Layer Forming Step>

In this step, amorphous silicon compound layers 4 are formed on the surfaces of objects to be covered. For the formation of amorphous silicon compound layers 4, a sol-gel method in which an organosilicon compound is hydrolyzed in a hydrophilic organic solvent and then subjected to dehydration condensation is preferably used.

Examples of the organosilicon compound include methyltriethoxysilane, methyltrimethoxysilane, tetraethoxysilane, tetramethoxysilane, tetraisopropoxysilane, and condensates thereof, γ-aminopropyltriethoxysilane, N-2-aminoethyl-3-aminopropyltriethoxysilane, and N-2-aminoethyl-3-aminopropylmethyldimethoxysilane. Examples of the hydrophilic organic solvent include methyl alcohol, ethyl alcohol, isopropyl alcohol, n-propyl alcohol, t-butyl alcohol, n-butyl alcohol, isobutyl alcohol, ethyl cellosolve, butyl cellosolve, propylene glycol monobutyl ether, dipropylene glycol monomethyl ether, propylene glycol monopropyl ether, and acetone.

By the above, amorphous silicon compound layers 4 are formed so as to be in contact with the surfaces of the objects to be covered. Amorphous silicon compound layers 4 formed in this manner are continuous layers having relatively smooth surfaces.

### <<Resin Layer Forming Step>>

As the outermost layers of coating films 2 that black aluminum pigment 10 includes, resin layers may be formed. In this case, the chemical resistance of black aluminum pigment 10 can be enhanced. The method of forming resin layers is not particularly limited, but a radical polymerization reaction is simple. Specifically, first, objects to be covered are dispersed in a nonpolar solvent, and a polymerizable monomer that is the constituent unit of a resin is added into the nonpolar solvent. Next, a polymerization initiator is added to radically polymerize the polymerizable monomer. Thus, resin layers obtained by the radical polymerization of the polymerizable monomer are formed on the objects to be covered.

Examples of preferred polymerizable monomers include reactive monomers having a carboxyl group and/or a phosphate group, tri- or higher polyfunctional acrylic ester monomers, and polymerizable monomers having a benzene nucleus as described above.

Examples of the reactive monomers having a carboxyl group and/or a phosphate group include acrylic acid, methacrylic acid, maleic acid, crotonic acid, itaconic acid, fumaric acid, 2-methacryloyloxyethyl acid phosphate, di-2-methacryloyloxyethyl acid phosphate, tri-2-methacryloyloxyethyl acid phosphate, 2-acryloyloxyethyl acid phosphate, di-2-acryloyloxyethyl acid phosphate, tri-2-acryloyloxyethyl acid phosphate, diphenyl-2-methacryloyloxyethyl acid phosphate, diphenyl-2-acryloyloxyethyl acid phosphate, dibutyl-2-methacryloyloxyethyl acid phosphate, dibutyl-2-acryloyloxyethyl acid phosphate, dioctyl-2-methacryloyloxyethyl acid phosphate, dioctyl-2-acryloyloxyethyl acid phosphate, 2-methacryloyloxypropyl acid phosphate, bis(2-chloroethyl)vinyl phosphonate, and diallyldibutyl phosphonosuccinate.

Examples of the tri- or higher polyfunctional acrylate monomers include trimethylolpropane triacrylate, trimethylolpropane trimethacrylate, tetramethylolpropane triacrylate, tetramethylolpropane tetraacrylate, tetramethylolpropane trimethacrylate, tetramethylolpropane tetramethacrylate, pentaerythritol triacrylate, pentaerythritol tetraacrylate, dipentaerythritol hexaacrylate, and ditrimethylolpropane tetraacrylate. These polyfunctional acrylate monomers have the effect of contributing to the three-dimensional crosslinking of the resin to insolubilize the resin layers in organic solvents and water.

Examples of the polymerizable monomers having a benzene nucleus include styrene, α-methylstyrene, vinyltoluene, divinylbenzene, phenyl vinyl ketone, phenyl vinyl ether, divinylbenzene monoxide phenoxyethyl acrylate, phenoxy-polyethylene glycol acrylate, and 2-hydroxy-3-phenoxypropyl acrylate. By copolymerizing these polymerizable monomers having a benzene nucleus, the barrier effect of the resin layer against chemicals improves, and the resin layers are particularly excellent in chemical resi stance.

Examples of the polymerization initiator include peroxides such as benzoyl peroxide, lauroyl peroxide, isobutyl peroxide, and methyl ethyl ketone peroxide, and azo compounds such as azobisisobutyronitrile.

As the nonpolar solvent, a hydrocarbon-based solvent is preferred. Specific examples include mineral spirits, petroleum benzine, solvent naphtha, isoparaffins, normal paraffins, benzene, toluene, xylene, cyclohexane, hexane, heptane, octane, chlorobenzene, trichlorobenzene, perchloroethylene, and trichloroethylene.

### <<Base layer Forming Step>>

As the innermost layers of coating films 2 that black aluminum pigment 10 includes, base layers may be formed. By forming the base layers on the surfaces of aluminum particles 1, the water resistance of aluminum particles 1 can be improved. In addition, when layer formation is performed on aluminum particles 1 by a sol-gel method, the layers by the sol-gel method grow easily with the base layers as starting points, due to the formation of the base layers on the surfaces of aluminum particles 1. The base layers can be good starting points, particularly of amorphous silicon compound layers 4 that grow by a sol-gel method.

The base layers are formed, for example, as follows. First, aluminum particles 1 that are objects to be covered, and a solution including a molybdenum compound and/or a phosphorus compound are stirred in a slurry state or a paste state, and then the mixture is heated, and thus base layers composed of at least one of an oxide, hydroxide, and hydrate of molybdenum and/or phosphorus are formed.

Black aluminum pigment 10 can be produced by carrying out the above-described steps in suitable order. For example, black aluminum pigment 10 shown in FIG. 2 can be produced by carrying out the preparation step, the titanium oxide layer forming step, and the amorphous silicon compound layer forming step in this order, and further the resin layer forming step may be carried out after the amorphous silicon compound layer forming step. In the titanium oxide layer forming step in this case, the second method is preferably used because when the first method is used, the dissolution of aluminum particles 1 is feared.

Black aluminum pigment 10 shown in FIG. 3 can be produced by carrying out the preparation step, the amorphous silicon compound layer forming step, and the titanium oxide layer forming step in this order, and further, the base layer forming step may be carried out between the preparation step and the amorphous silicon compound layer forming step, and the resin layer forming step may be carried out after the titanium oxide layer forming step. In the titanium oxide layer forming step in this case, the first method is preferably used. In this case, titanium oxide layers 3 can be formed in water, and therefore the production cost can be reduced compared with the second method using a hydrophilic organic solvent.

Black aluminum pigment 10 shown in FIG. 4 can be produced by carrying out the preparation step, the amorphous silicon compound layer forming step, the titanium oxide layer forming step, and the amorphous silicon compound layer forming step in this order, and further, the base layer forming step may be carried out between the preparation step and the amorphous silicon compound layer forming step, and the resin layer forming step may be carried out after the second amorphous silicon compound layer forming step. In the titanium oxide layer forming step in this case, the first method is preferably used for the above-described reason.

### [Resin Composition]

A resin composition according to this embodiment is a resin composition including the above-described black aluminum pigment 10. Such resin compositions include, for example, paints, coatings formed from these paints, inks, printed materials obtained by printing these inks, and cosmetics.

The resin composition according to this embodiment can be applied to both an organic solvent type (oily) and aqueous. Particularly when amorphous silicon compound layer 4 is present outside titanium oxide layer 3 as shown in FIG. 2 and FIG. 4, the resin composition has high resistance to aqueous solvents and therefore can be applied to aqueous paints. As examples of the resin composition, a paint and a cosmetic will be described in detail below.

### <Paint>

A paint according to this embodiment is a paint including the above-described black aluminum pigment 10. The paint can also include, in addition to black aluminum pigment 10, another pigment, an additive, a resin, a solvent, and the like.

Examples of the above another pigment can include organic color pigments, inorganic color pigments, extender pigments, and color pigments such as plate-like iron oxide. Examples of the additive can include pigment dispersing agents, antifoaming agents, antisettling agents, and curing catalysts. Examples of the resin can include epoxy resins, polyester resins, alkyd resins, acrylic resins, acrylic silicone resins, vinyl resins, silicon resins (inorganic binders), polyamide resins, polyamide-imide resins, melamine resins, fluororesins, synthetic resin emulsions, boiled oils, chlorinated rubbers, natural resins and amino resins, phenolic resins, and polyisocyanate resins. Examples of the solvent can include organic solvents such as alcohol-based, glycol-based, ketone-based, ester-based, ether-based, aromatic, and hydrocarbon-based solvents, and water.

The amount of black aluminum pigment 10 blended in the paint is appropriately changed by the required design properties but is preferably 0.1 to 50 parts by mass, more preferably 1 to 35 parts by mass, based on 100 parts by mass of the paint resin. When this amount blended is 0.1 parts by mass or more, the decorative effect is good. When this amount blended is 50 parts by mass or less, the adhesiveness, weather resistance, corrosion resistance, adhesive strength, and the like of the paint are good.

### [Cosmetic]

A cosmetic according to this embodiment is a cosmetic including the above-described black aluminum pigment 10. Examples of cosmetics to which such a cosmetic is applied include makeup cosmetics (lipsticks, foundations, blushers, eye shadows, nail enamels, and the like), hair cosmetics (hair gels, hair waxes, hair treatments, shampoos, hair manicure gels, and the like), and basic cosmetics (base creams and the like).

Conventionally, in order to provide a glossy feeling and a brilliant feeling to cosmetics, pearl pigments, aluminum pigments, and the like are used. But the pearl pigments are poor in hiding properties, and the aluminum pigments provide a gray color, and therefore a vivid black color tone has tended not to be obtained even if another color pigment is blended to form a cosmetic. Further, the aluminum pigments react easily with water and therefore have not been able to be applied to cosmetics containing water.

In contrast to this, the above-described black aluminum pigment 10 can have a metallic appearance and provide a black color tone. In addition, black aluminum pigment 10 has amorphous silicon compound layer 4 in coating film 2 and therefore can also be applied to cosmetics containing water. Particularly, when amorphous silicon compound layer 4 is present outside titanium oxide layer 3 as shown in FIG. 2 and FIG. 4, black aluminum pigment 10 has high resistance to aqueous solvents and therefore can be preferably applied to cosmetics.

The cosmetic according to this embodiment can include, in addition to black aluminum pigment 10, oil, a surfactant, a moisturizer, a polyhydric alcohol, a watersoluble polymer, a film-forming agent, a water-insoluble polymer, a polymer emulsion, a powder, a pigment, a dye, a lake, a lower alcohol, an ultraviolet absorbing agent, vitamins, an antioxidant, an antimicrobial agent, a perfume, water, and the like.

### [Action and Effect]

Black aluminum pigment 10 according to this embodiment can have high commercial utility value as a brilliant black pigment. The reason is as follows.

In black aluminum pigment 10, coating films 2 including titanium oxide layers 3 and amorphous silicon compound layers 4 are formed on aluminum particles 1. When amorphous silicon compound layers 4 are interposed between aluminum particles 1 and titanium oxide layers 3, titanium oxide layers 3 can be easily formed by carrying out the above-described (1) hydrolysis treatment and (2) reduction treatment. When amorphous silicon compound layers 4 are not interposed between aluminum particles 1 and titanium oxide layers 3, titanium oxide layers 3 can be easily formed by carrying out the above-described (3) sol-gel treatment and (4) reduction treatment.

In both of the above cases, homogeneous titanium oxide layers 3 can be formed, and unintended dissolution of aluminum particles 1 that are base materials can be sufficiently suppressed. This is because in the methods, the positions of amorphous silicon compound layers 4 in coating films 2 are suitably disposed. In addition, amorphous silicon compound layers 4 not only suppress the dissolution of aluminum particles 1 during the production process but can also provide high water resistance to black aluminum pigment 10. Therefore, black aluminum pigment 10 can be applied to both oily and aqueous compositions by appropriately changing the layered structures of coating films 2.

In this manner, black aluminum pigment 10 according to this embodiment can have high water resistance, exhibit a high metallic appearance, and provide a black color. Therefore, black aluminum pigment 10 according to this embodiment can have high commercial utility value as a brilliant black pigment. Particularly, as described above, black aluminum pigment 10 according to this embodiment can be preferably applied to resin compositions such as paints and cosmetics.

### EXAMPLES

The present invention will be more specifically described below by giving Examples and Comparative Examples, but the present invention is not limited to these. The black aluminum pigments of the Examples and the aluminum pigments of the Comparative Examples may be collectively described below as "(black) aluminum pigments".

### <Methods of Measuring and Evaluating Various Types of Characteristics>

### <<Measurement of Average Particle Diameter of Aluminum Particles>>

First, about 0.1 g of aluminum particles were introduced into 20 g of ethanol and dispersed by a glass rod, and then sample collection was performed using a dropper. After the sample collection, the sample was rapidly introduced into a laser diffraction type particle size measuring instrument ("Microtrac MT3000II", manufactured by MicrotracBEL Corp.) and dispersed by an ultrasonic dispersing machine attached to the apparatus, and then measurement was carried out. The circulating solvent in the apparatus was ethanol that was the same as the dispersion medium, the output of ultrasonic dispersion was 40 W, and 1 min ultrasonic irradiation was carried out. Then, the measurement of particle size distribution was immediately carried out.

### <<Measurement of Thicknesses of Layers>>

A test piece for observation was made, and the thicknesses of the layers were calculated according to the above-described method. For observation, a TEM ("JEM-ARM200F", manufactured by JEOL Ltd.) was used, and the observation magnification was 50000 to 100000x. The test piece for observation was made as follows.

First, 17.0 g of an adjusting clear ("Nax Admila 280", manufactured by Nippon Paint Co., Ltd.) and 3.0 g of a binder ("Nax Admila 901", manufactured by Nippon Paint Co., Ltd.) were mixed in a beaker using a glass rod. Then, 1.2 g of an (black) aluminum pigment as solids was introduced into the mixed liquid, and the mixture was stirred by a glass rod. Further, the (black) aluminum pigment was dispersed using a stirring and defoaming apparatus to obtain a (black) aluminum pigment-dispersed paint. A PET film was coated with the (black) aluminum pigment-dispersed paint using an applicator (9 mil), allowed to stand at room temperature for 20 min, and then dried at 80°C for 20 min to form a coating on the PET film. The formed coating was peeled from the PET film, and this was sliced using a focused ion beam apparatus, and thus a test piece for observation including a cross section of the (black) aluminum pigment was made. The above method of making a test piece for observation for a TEM is one example, and the method is not limited.

### <<Confirmation of Composition of Amorphous Silicon Compound Layer>>

The composition of an amorphous silicon compound layer was confirmed by the simultaneous detection of the signals of Si and O from the surface of a (black) aluminum pigment, using an EDX ("EX-23000BU", manufactured by JEOL Ltd.). In addition, for the amorphous silicon compound layer, the presence or absence of diffraction peaks derived from silicon oxide was confirmed using a powder diffraction X-ray analysis apparatus ("Ultima IV", manufactured by Rigaku Corporation). When the diffraction peaks were not confirmed, the layer was regarded as amorphous.

### <<Measurement of Composition of Titanium Oxide Layer>>

A powder diffraction X-ray analysis apparatus ("Ultima IV", manufactured by Rigaku Corporation) and integrated powder X-ray analysis software ("PDXL 2.7", manufactured by Rigaku Corporation) were used. The confirmation of the composition of lower titanium oxide was performed under the conditions of a tube voltage of 40 kV and a tube current of 20 mA using an RIR method.

### <<Color Tone Evaluation>>

The evaluation of the color tone of a (black) aluminum pigment was performed by the Hunter Lab color system using a colorimetric color difference meter ("TC-8600A", Tokyo Denshoku Co., Ltd.). Colorimetry was performed by a powder cell method, and 1 g of a (black) aluminum pigment was added to a glass cell, and a surface pressure of 25.5 g/cm² was applied to form a measurement sample. For the conditions of the colorimetry, D/0° was set, and a C light source was adopted.

### <<Water Resistance Evaluation>>

First, a mixed liquid of 90 g of ion-exchanged water and 90 g of butyl cellosolve was prepared, and 5.0 g of an aluminum pigment was introduced thereinto and dispersed by a glass rod. Next, the pH at 25°C was adjusted at 11 ± 0.5 using 2-dimethylaminoethanol. Then, 180 g of the mixed liquid was enclosed in a dedicated container for gas generation measurement, and maintained for 48 h while being kept at 40°C. The amount of hydrogen gas generated during this time was measured to evaluate water resistance. The hydrogen gas is a gas generated by the corrosion and dissolution of aluminum.

### <Example 1, not in accordance with the invention>

An aluminum pigment in which an amorphous silicon oxide layer and a titanium oxide layer were layered in this order on an aluminum particle was produced as follows.

### <<Preparation Step>>

As flaky aluminum particles, "5422NS" (solid content 75% by mass, D50: 19 µm) manufactured by Toyo Aluminium K.K. was prepared. 9 g of hydrogen peroxide water (hydrogen peroxide 30% by mass) was dissolved in 1500 g of isopropyl alcohol (hereinafter abbreviated as IPA) in a 3 L round bottom flask equipped with a stirrer, and further 133.3 g of the aluminum particles (that is, 100 g as aluminum) were added, followed by stirring and mixing at 75°C for 1 h to obtain a slurry.

### <<Amorphous Silicon Oxide Layer Layering Step>>

80 g of ion-exchanged water was added to the slurry, and the pH value of the slurry was adjusted at about 9.0 while ammonia water was further added. A solution of 100 g of tetraethoxysilane dissolved in 100 g of IPA was gradually dropped into the pH-adjusted slurry, further followed by stirring and mixing at 75°C for 2 h. Then, the slurry was subjected to solid-liquid separation by a filter, and then the solids were dried in an oven at 100°C. Thus, aluminum particles in which an amorphous silicon oxide layer was formed on the surface of an aluminum particle (hereinafter described as "aluminum particles (A)") were obtained.

### <<Titanium Oxide Layer Forming Step>>

### (Hydrolysis Treatment)

100 g of the aluminum particles (A) were introduced into 2000 g of ion-exchanged water, and the mixture was stirred to form a slurry. This was heated to 75°C while being stirred. Next, the total amount of a solution of 2 g of tin chloride-pentahydrate dissolved in 8 g of ion-exchanged water was added to the slurry. Then, the pH of the slurry was adjusted at 1.5 using a 10% hydrochloric acid aqueous solution. Next, a titanium tetrachloride aqueous solution (containing 22% by mass of TiO₂) was added to the slurry after the pH adjustment at a rate of 1 mL/min, and continuously added until the slurry provided a light green color. At this time, a 30% by mass sodium hydroxide aqueous solution was simultaneously added to control fluctuations in pH within the range of 1.4 to 1.6. Then, the slurry was neutralized using a 30% by mass sodium hydroxide aqueous solution until the pH of the slurry reached 7.0. Then, the slurry was cooled to 40°C, and solid-liquid separation and washing with ion-exchanged water were performed. After the water washing, drying was performed in an oven at 80°C until the solid content reached 99% or more. Thus, aluminum particles in which an amorphous silicon compound layer and a titanium dioxide layer were layered in this order on the surface of an aluminum particle (hereinafter described as "aluminum particles (B)") were obtained.

### (Reduction Treatment)

10.0 g of sodium borohydride as a reduction aid was added to 100 g of the aluminum particles (B), and firing at 600°C for 3 h was performed while nitrogen gas was introduced at 100 ml/min. After the firing, water washing treatment and dewatering treatment were performed, and then drying was performed at 70°C. Thus, a black aluminum pigment in which an amorphous silicon compound layer and a titanium oxide layer were layered in this order on the surface of an aluminum particle was produced.

### <Example 2, not in accordance with the invention>

A black aluminum pigment was produced by the same method as Example 1 except that in the reduction treatment, 7.0 g of sodium borohydride was added to 100 g of the aluminum particles (B).

### <Example 3, not in accordance with the invention>

A black aluminum pigment was produced by the same method as Example 1 except that in the reduction treatment, 15.0 g of sodium borohydride was added to 100 g of the aluminum particles (B).

### <Example 4, not in accordance with the invention>

A black aluminum pigment was produced by the same method as Example 1 except that in the reduction treatment, a mixed gas in which hydrogen:nitrogen was mixed at 2:1 was used instead of nitrogen gas.

### <Comparative Example 1>

An aluminum pigment was produced by the same method as Example 1 except that in the reduction treatment, 4.0 g of sodium borohydride was added to 100 g of the aluminum particles (B).

### <Example 5, in accordance with the invention >

An aluminum pigment in which a titanium oxide layer and an amorphous silicon oxide layer were layered in this order on an aluminum particle was produced as follows.

### <<Titanium Oxide Layer Forming Step>

### (Sol-Gel Treatment)

50 g of a butyl titanate dimer ("ORGATIX TA-23", manufactured by Matsumoto Fine Chemical Co., Ltd.) was added to a slurry obtained through the same preparation step as Example 1, and the mixture was stirred for 10 min. Further, a solution of 50 g of 25% by mass ammonia water dissolved in 450 g of IPA was added over 2 h, and further, stirring was performed for 2 h. Then, the slurry was filtered, and then washing with IPA was repeated, and then the solids were recovered. The recovered solids were dried at 140°C for 3 h. Thus, aluminum particles in which a titanium dioxide layer was formed on the surface of an aluminum particle (hereinafter described as "aluminum particles (C)") were obtained.

### (Reduction Treatment)

Reduction treatment was carried out on 100 g of the aluminum particles (C) by the same method as Example 1. By the above, aluminum particles in which a titanium oxide layer was formed on the surface of an aluminum particle (hereinafter described as "aluminum particles (D)") were obtained.

### <<Amorphous Silicon Compound Layer Forming Step>>

100 g of the aluminum particles (D) were dispersed in 1000 g of IPA, followed by stirring and mixing at 75°C for 30 min to obtain a slurry. Further, 80 g of ion-exchanged water was added to the obtained slurry, and the pH value of the slurry was adjusted at about 10.0 while ammonia water was further added. A solution of 50 g of tetraethoxysilane dissolved in 50 g of IPA was gradually dropped into the pH-adjusted slurry, further followed by stirring and mixing at 75°C for 2 h. Then, the slurry was subjected to solid-liquid separation by a filter, and then drying was performed in an oven at 100°C. By the above, an aluminum pigment in which a titanium oxide layer and an amorphous silicon compound layer were layered in this order on the surface of an aluminum particle was produced.

### <Comparative Example 2>

An aluminum pigment was produced by the same method as Example 5 except that the amorphous silicon compound layer was not formed.

**[Table 1]**

| | Example 1 | Example 2 | Example 3 | Example 4 | Example 5 | Comparative Example 1 | Comparative Example 2 |
|---|---|---|---|---|---|---|---|
| Thickness of amorphous silicon oxide layer (nm) | 90 | - | - | - | 60 | - | None |
| Thickness of titanium oxide layer (nm) | 250 | - | - | - | 100 | - | - |
| Amount of hydrogen gas generated (ml) | 3 | - | - | - | 0 | - | Impossible |
| L* value | 16.3 | 19.1 | 13.4 | 18.5 | 18.3 | 33.1 | 17.5 |
| X value of TiOₓ | 1.53 | 1.86 | 1.19 | 1.71 | 1.62 | 1.92 | 1.62 |
| Appearance color | Black | Bluish black | Reddish black | Black | Black | Reddish brown | Black |

### <Consideration>

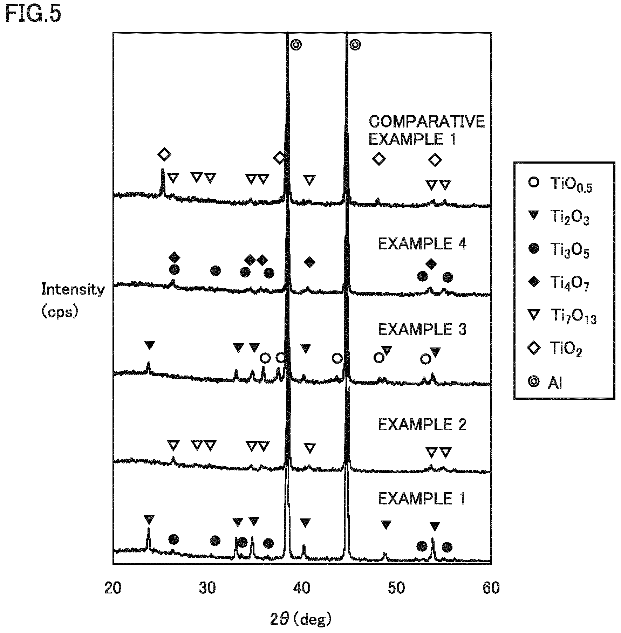

The results of Examples 1 to 5 and Comparative Examples 1 to 2 are shown in Table 1. The analysis results of powder X-ray diffraction for the aluminum pigments of Examples 1 to 4 and Comparative Example 1 are shown in FIG. 5. The measurement of the thickness of the amorphous silicon compound layer and the thickness of the titanium oxide layer and the water resistance evaluation were carried out only on Example 1, Example 5, and Comparative Example 2. "-" in Table 1 means that measurement is not carried out, "None" means that the layer is not present, and "Impossible" means that the generation of hydrogen gas was significant, and measurement was impossible.

With reference to Table 1, for the X value of the composition of the titanium oxide layer, Example 1 to Example 5 satisfied TiOₓ (0.50 ≤ x ≤ 1.90), and Comparative Example 1 did not satisfy TiOₓ (0.50 ≤ x ≤ 1.90). Regarding the color tone, regarding Examples 1 to 5 that satisfied TiOₓ (0.50 ≤ x ≤ 1.90), it was confirmed by visual inspection that Examples 1 to 5 provided a black color. Example 2 was black tinged with blue by visual inspection, and Example 3 was black tinged with red by visual inspection, but the L* value was as low as 20 or less, and Example 2 and Example 3 were determined as black. In contrast to this, Comparative Example 1 was reddish brown in visual inspection. In addition, the L* value of the pigment was also 33.1, that is, the lightness was high, and the pigment could not be determined as black.

Regarding the water resistance evaluation, the amount of hydrogen gas generated in Example 1 was sufficiently low, and it was confirmed that Example 1 had high water resistance. Particularly in Example 5, hydrogen gas was not generated. From this, it was found that when an amorphous silicon compound layer was formed outside a titanium oxide layer in a black aluminum pigment, the black aluminum pigment had particularly high water resistance. This is considered to be because an amorphous silicon compound layer formed inside a titanium oxide layer may have defects accompanying firing during titanium oxide layer formation, whereas an amorphous silicon compound layer formed outside a titanium oxide layer does not have such defects and therefore can consequently exhibit significantly excellent water resistance. In the aluminum pigment of Comparative Example 2 that did not have an amorphous silicon compound layer, hydrogen was excessively generated, and therefore the amount of hydrogen generated could not be measured.

With reference to FIG. 5, TiO_{0.5} was included in the titanium oxide layer of Example 3. For titanium oxide, it is known that as the oxygen deficiency increases, light is easily absorbed in titanium oxide, and thus the titanium oxide provides a black color. From the results of Example 3, even when the X value of TiOₓ was 0.50, the titanium oxide layer was regarded as providing a black color.

A use example and production examples of the present invention are shown below.

### <Use Example 1: Black Base Paint>

100 Parts by mass of a composition consisting of a composition A was diluted with the following thinner to a viscosity suitable for spray coating (12 to 15 s by a Ford cup #4), and then a base coat layer was formed by spray coating. The formed base coat layer was coated with the following clear paint and then air-dried at room temperature for 20 min, and then baking was performed at 130°C for 30 min. The obtained coating provided a brilliant black color excellent in hiding power. For the black aluminum pigment, the black aluminum pigment of Example 5 was used.

### (Composition A)

| | |
|---|---|
| ACRYDIC 47-712 | 64 parts by mass |
| SUPER BECKAMINE G821-60 | 27 parts by mass |
| black aluminum pigment | 9 parts by mass. |

### (Thinner)

| | |
|---|---|
| ethyl acetate | 50 parts by mass |
| toluene | 30 parts by mass |
| n-butanol | 10 parts by mass |
| Solvesso #150 | 40 parts by mass. |

### (Clear Paint)

| | |
|---|---|
| ACRYDIC 44-179 | 14 parts by mass |
| SUPER BECKAMINE L117-60 | 6 parts by mass |
| toluene | 4 parts by mass |
| MIBK | 4 parts by mass |
| butyl cellosolve | 3 parts by mass. |

The cosmetics of Production Example 1 and Production Example 2 were produced below. The produced cosmetics provided an excellent metallic appearance and a black color. For the black aluminum pigment, the black aluminum pigment of Example 5 was used.

### <Production Example 1: Hair Gel>

| | |
|---|---|
| carboxyvinyl polymer | 5.0 parts by mass |
| ethyl alcohol | 2.0 parts by mass |
| PEG1500 | 1.0 part by mass |
| aminomethyl propanol | 1.5 parts by mass |
| methylparaben | 0.1 parts by mass |
| black aluminum pigment | 7.0 parts by mass |
| purified water | 83.4 parts by mass. |

### <Production Example 2: Nail Enamel>

| | |
|---|---|
| nitrocellulose (viscosity 1/2 s) | 10.0 parts by mass |
| alkyd resin | 10.0 parts by mass |
| acetotributyl citrate | 5.0 parts by mass |
| ethyl acetate | 25.0 parts by mass |
| butyl acetate | 40.0 parts by mass |
| ethyl alcohol | 5.0 parts by mass |
| black aluminum pigment | 5.0 parts by mass. |

The embodiments and the Examples disclosed this time should be considered as illustrative in all points and not restrictive.

### REFERENCE SIGNS LIST

1 aluminum particle, 2 coating film, 3 titanium oxide layer, 4 amorphous silicon compound layer, 10 black aluminum pigment

## Claims

1. A black aluminum pigment (10) comprising:
a flaky aluminum particle (1); and
a coating film (2) that covers the aluminum particle (1), wherein
the coating film (2) comprises a titanium oxide layer (3) and an amorphous silicon compound layer (4) wherein the titanium oxide layer (3) and the amorphous silicon compound layer (4) are layered in this order,
the titanium oxide layer (3) has a composition that satisfies TiOₓ (0.50 ≤ x ≤ 1.90), and
the amorphous silicon compound layer (4) is composed of at least one of silicon oxide, silicon hydroxide, and silicon hydrate.

2. The black aluminum pigment (10) according to claim 1, wherein the titanium oxide layer (3) has a thickness of 50 nm or more and 1000 nm or less.

3. The black aluminum pigment (10) according to claim 1 or 2, wherein the amorphous silicon compound layer (4) has a thickness of 10 nm or more and 1000 nm or less.

4. A method of producing the black aluminum pigment (10) according to any one of claim 1 to claim 3, comprising:
preparing an aluminum particle (1); and
forming a coating film (2) on the aluminum particle (1), wherein
forming the coating film (2) has forming a titanium oxide layer (3), and forming an amorphous silicon compound layer (4),
when the amorphous silicon compound layer (4) is interposed between the aluminum particle (1) and the titanium oxide layer (3), the titanium oxide layer (3) is formed by subjecting to reduction treatment a titanium dioxide layer formed by hydrolysis treatment, or
when the amorphous silicon compound layer (4) is not interposed between the aluminum particle (1) and the titanium oxide layer (3), the titanium oxide layer (3) is formed by subjecting to reduction treatment a titanium dioxide layer formed by sol-gel treatment.

## Patentansprüche

1. Schwarzes Aluminiumpigment (10), umfassend:
ein plättchenförmiges Aluminiumpartikel (1) und
einen Beschichtungsfilm (2), der das Aluminiumpartikel (1) bedeckt, wobei
der Beschichtungsfilm (2) eine Titanoxidschicht (3) und eine amorphe Siliziumverbindungsschicht (4) umfasst, wobei die Titanoxidschicht (3) und die amorphe Siliziumverbindungsschicht (4) in dieser Reihenfolge als Schichten angeordnet sind,
die Titanoxidschicht (3) eine Zusammensetzung aufweist, die TiOₓ (0,50 ≤ x ≤ 1,90) erfüllt, und
die Schicht aus einer amorphen Siliziumverbindung (4) aus wenigstens einem von Siliziumoxid, Siliziumhydroxid und/oder Siliziumhydrat aufgebaut ist.

2. Schwarzes Aluminiumpigment (10) nach Anspruch 1, wobei die Titanoxidschicht (3) eine Dicke von wenigstens 50 nm und höchstens 1000 nm aufweist.

3. Schwarzes Aluminiumpigment (10) nach Anspruch 1 oder 2, wobei die amorphe Siliziumverbindungsschicht (4) eine Dicke von wenigstens 10 nm und höchstens 1000 nm aufweist.

4. Verfahren zur Herstellung des schwarzen Aluminiumpigments (10) nach einem der Ansprüche 1 bis 3, umfassend:
Herstellen eines Aluminiumpartikels (1) und
Ausbilden eines Beschichtungsfilms (2) auf dem Aluminiumpartikel (1), wobei
das Ausbilden des Beschichtungsfilms (2) ein Ausbilden einer Titanoxidschicht (3) und ein Ausbilden einer amorphen Siliziumverbindungsschicht (4) umfasst,
wobei wenn die amorphe Siliziumverbindungsschicht (4) zwischen dem Aluminiumpartikel (1) und der Titanoxidschicht (3) angeordnet wird, die Titanoxidschicht (3) dadurch ausgebildet wird, dass eine Titandioxidschicht, die durch eine Hydrolysebehandlung entstanden ist, einer Reduktionsbehandlung unterzogen wird, oder,
wenn die amorphe Siliziumverbindungsschicht (4) nicht zwischen dem Aluminiumpartikel (1) und der Titanoxidschicht (3) angeordnet wird, die Titanoxidschicht (3) dadurch ausgebildet wird, dass eine Titandioxidschicht, die durch eine Sol-Gel-Behandlung entstanden ist, einer Reduktionsbehandlung unterzogen wird.

## Revendications

1. Pigment d'aluminium noir (10) comprenant :
une particule d'aluminium feuilletée (1) et
un film de revêtement (2) qui recouvre la particule d'aluminium (1), dans lequel
le film de revêtement (2) comprend une couche d'oxyde de titane (3) et une couche de composé de silicium amorphe (4), la couche d'oxyde de titane (3) et la couche de composé de silicium amorphe (4) étant couchées dans cet ordre,
la couche d'oxyde de titane (3) a une composition qui satisfait TiOx (0,50 ≤ x ≤ 1,90) et
la couche de composé de silicium amorphe (4) est composée d'au moins d'un oxyde de silicium, d'un hydroxyde de silicium, et d'un hydrate de silicium.

2. Pigment d'aluminium noir (10) selon la revendication 1, dans lequel la couche d'oxyde de titane (3) a une épaisseur de 50 nm ou plus et de 1 000 nm ou moins.

3. Pigment d'aluminium noir (10) selon la revendication 1 ou 2, dans lequel la couche de composé de silicium amorphe (4) a une épaisseur de 10 nm ou plus et de 1 000 nm ou moins.

4. Procédé de production du pigment d'aluminium noir (10) selon l'une quelconque des revendications 1 à 3, comprenant les étapes consistant à :
préparer une particule d'aluminium (1) ; et
former un film de revêtement (2) sur la particule d'aluminium (1),
la formation du film de revêtement (2) ayant la formation d'une couche d'oxyde de titane (3) et la formation d'une couche de composé de silicium amorphe (4),
lorsque la couche de composé de silicium amorphe (4) est interposée entre la particule d'aluminium (1) et la couche d'oxyde de titane (3), la couche d'oxyde de titane (3) est formée en soumettant à un traitement de réduction une couche de dioxyde de titane formée par traitement par hydrolyse ou,
lorsque la couche de composé de silicium amorphe (4) n'est pas interposée entre la particule d'aluminium (1) et la couche d'oxyde de titane (3), la couche d'oxyde de titane (3) est formée en soumettant à un traitement de réduction une couche de dioxyde de titane formée par un traitement sol-gel.
